Europäisches Patentamt

European Patent Office

Office européen des brevets

(19)

(11) Numéro de publication : **0 169 868**
**B1**

(12) **FASCICULE DE BREVET EUROPÉEN**

(45) Date de publication du fascicule du brevet :
11.11.87

(51) Int. Cl.⁴ : **A 41 B 13/02**

(21) Numéro de dépôt : 85900683.5

(22) Date de dépôt : 25.01.85

(86) Numéro de dépôt international :
**PCT/FR 85/00014**

(87) Numéro de publication internationale :
**WO/8503205 (01.08.85 Gazette 85/17)**

(54) **ARTICLE D'HYGIENE A JETER AVEC CEINTURE AMOVIBLE.**

(30) Priorité : 27.01.84 FR 8401333
18.05.84 FR 8407713
06.11.84 FR 8416844

(43) Date de publication de la demande :
**05.02.86 Bulletin 86/06**

(45) Mention de la délivrance du brevet :
**11.11.87 Bulletin 87/46**

(84) Etats contractants désignés :
**AT BE CH DE FR GB LI LU NL SE**

(56) Documents cités :
**DE-A- 1 435 900**
**FR-A- 2 112 863**
**US-A- 2 509 674**
**US-A- 2 695 025**
**US-A- 3 089 494**
**US-A- 3 150 664**

(73) Titulaire : **BEGHIN-SAY SOCIETE ANONYME**

**F-59239 Thumeries (FR)**

(72) Inventeur : **PIGNEUL, Raymond**
**2, rue des Vosges**
**F-68320 Durrenentzen (FR)**
Inventeur : **PANNIER, Dominique**
**9, rue des Iris**
**F-68000 Colmar (FR)**
Inventeur : **TRESCA, Benoit**
**6, rue Stockemeyer**
**F-68000 Colmar (FR)**

(74) Mandataire : **Quéré, Jean Pierre**
**BEGHIN-SAY Service Propriété Industrielle 54, Avenue Hoche**
**F-75008 Paris (FR)**

## Description

L'invention a pour objet un article d'hygiène à jeter absorbant les liquides pour bébés ou pour adultes et a plus particulièrement pour but de proposer de nouveaux moyens et un agencement permettant d'assurer le maintien dudit article sur l'utilisateur grâce à une ceinture amovible.

En général, un tel article est essentiellement constitué d'un matelas absorbant formé d'une structure stratifiée comprenant une nappe absorbante en mousse de cellulose, dans laquelle sont, éventuellement, incorporées des poudres absorbant plusieurs fois leur poids en liquide, recouverte sur la face destinée à être en contact avec la peau de l'utilisateur d'une feuille perméable aux liquides au moins dans la partie centrale et sur la face opposée, d'un film de polyéthylène, les deux feuilles étant partiellement ou totalement soudées l'une à l'autre le long de leurs bords. Par ailleurs, des moyens de fixation sont prévus au niveau des zones de tronc, de manière à assurer le maintien de l'article sur l'utilisateur.

On appelle « zone de tronc » les zones de l'article qui sont destinées à être, en position, en contact avec l'abdomen et le dos de l'utilisateur.

Parmi les nombreux moyens de fixation déjà proposés (épingles de sûreté, boutons-boutonnières, crochets-ouvertures) les adhésifs, et notamment ceux sensibles à la pression, sont les plus communément employés.

Les systèmes de fixation adhésifs présentent, toutefois, l'inconvénient de ne pas être « repositionnables » une fois mis en place, à moins d'adopter des attaches de structure complexe, constituées de plusieurs couches superposées comme cela est décrit dans le brevet US-A-3 951 149. Ces attaches sont, d'une part, très onéreuses et, d'autre part, très difficiles à fabriquer industriellement, du fait des diverses qualités d'adhésifs qu'il est nécessaire d'apposer successivement sur les différents supports.

Dans la présente demande, les moyens de fixation sont du type « boucles-griffes », mieux connus sous le nom de marque « Velcro » (R).

De nombreux documents décrivent déjà des articles à jeter dont les moyens de fixation sont constitués par des bandes « Velcro » (R).

Le brevet GB-A-1 428 572 décrit un vêtement pour bébés, sur la face interne duquel on place un matelas absorbant et dont les bords d'une zone de tronc externe, rabattus sur l'autre zone de tronc entourant le ventre du bébé sont maintenus au moyen de bandes « Velcro » (R) situées sur les deux zones de tronc. Un dispositif similaire est également décrit dans les brevets US-A-3 081 772 et US-A-3 141 461 (figure 11).

Dans le brevet US-A-4 402 690 est décrit un dispositif dans lequel les bords d'une zone de tronc interne sont pourvus d'éléments d'attache « Velcro » (R) tandis que les bords de l'autre zone de tronc externe sont pourvus des éléments d'attache complémentaires.

Les articles, tous à usage multiple, décrits dans ces documents présentent l'inconvénient de nécessiter par change la présence de quatre éléments « Velcro » (R) de grandes dimensions, deux sur la face interne, deux sur la face externe. Une telle disposition, appliquée à des articles à usage unique tels que ceux objets de l'invention en grèverait considérablement le prix de revient. En effet, ces éléments sont onéreux mais il ne serait pas pour autant possible d'en trop limiter les dimensions, faute d'assurer une amplitude d'ajustement suffisante, de plus le fait de devoir déposer les éléments sur les deux faces compliquerait les installations de fabrication qui devraient comporter un double poste de pose des éléments, un de chaque côté de la bande de défilement.

La même critique peut être émise à propos de l'article décrit dans le brevet US-A-3 359 980. Cet article est muni de deux bandes « Velcro » (R) sur les bords de la face interne d'une zone de tronc et d'une bande « Velcro » complémentaire sur pratiquement toute la largeur de la face externe de l'autre zone de tronc.

Afin de remédier à ces inconvénients majeurs, il a été proposé de relier les bords des zones de tronc arrière d'un change, à ceux des zones avant situées sur l'abdomen de l'utilisateur, par deux demi-ceintures amovibles fixées l'une à l'autre par deux éléments « Velcro » (R) complémentaires et à leurs bords respectifs par des pinces à griffes. Cet article est décrit dans le brevet US-A-4 158 906. Outre le danger et l'inconfort que peuvent représenter pour un bébé ou même un adulte des mâchoires acérées, ce système présente d'autres inconvénients importants. Lorsque les deux mâchoires sont fixées de chaque côté aux zones arrière de l'article, l'effort de traction appliqué à ce niveau a tendance à déchirer les bords du change : les feuilles recouvrant le matelas absorbant sont minces, facilement déformables et déchirables, propriétés qui sont, par ailleurs, un gage de confort pour l'utilisateur. Ainsi le mode de fixation proposé par ce brevet n'est que très difficilement utilisable pour les articles à jeter. En outre, la partie avant, non maintenue, a tendance à glisser.

On connaît également, par le brevet WO 83/04163, une couche formée d'une partie avant pourvue de points de connexion au travers desquels on peut faire passer un matériau filamenteux, la partie arrière étant pourvue de deux demi-longueurs de matériau filamenteux fixées au bord supérieur opposé arrière de celle-ci de manière à maintenir la couche en place par le passage des deux demi-longueurs au travers des points de connexion et le nouage des extrémités des matériaux filamenteux autour de l'abdomen de l'utilisateur.

Cette couche présente certains inconvénients. En effet, les points de connexion compliquent singulièrement la fabrication de la couche. Par ailleurs, ils limitent le réglage de la couche sur l'utilisateur à une seule position, sinon il faut prévoir d'autres points de connexion. Dans le cas d'un bébé, le passage des matériaux filamenteux au travers des points de connexion réclame de la part d'une mère l'usage des deux mains, ce qui est dangereux car un bébé a tendance à gigoter.

L'intérêt de l'invention qui va être décrite ci-après réside d'abord dans la simplicité et l'efficacité du mode de fixation proposé. Elle permet, en outre, un gain très important sur le plan économique du fait que la ceinture amovible est réutilisable pour de nombreux articles. Par ailleurs, la fabrication industrielle de l'article s'en trouve simplifiée.

Selon l'invention, l'article à jeter constitué :

a) d'un matelas absorbant de forme sensiblement rectangulaire comportant deux zones de tronc et une zone d'entre-jambe, recouvert sur sa face externe d'une feuille imperméable aux liquides, telle qu'une feuille de polyéthylène et sur sa face interne d'une feuille perméable aux liquides au moins au niveau de la zone centrale, ces deux feuilles étant partiellement ou totalement soudées entre elles le long de leurs bords longitudinaux, un premier élément d'attache étant apposé à proximité de chaque bord longitudinal sur la face externe au niveau d'une zone de tronc

b) d'un moyen de fixation associé de manière amovible, comprenant une ceinture pourvue à chaque extrémité d'un second élément d'attache complémentaire du premier élément d'attache

est caractérisé en ce que, l'autre zone de tronc externe et/ou au moins une zone de la ceinture venant en contact avec cette zone de tronc externe est constituée de manière à posséder un coeffficient de friction suffisant tel que, en position, l'article soit maintenu d'une part par la coopération des premiers éléments d'attache avec les seconds éléments d'attache, d'autre part par le frottement, l'une sur l'autre de la zone de tronc et de ladite zone de ceinture.

Le préambule de la revendication 1 (a) et (b) est connu du document US-A-2 695 025.

Dans cette description les qualificatifs « longitudinal » et « latéral » sont définis par rapport à l'article sans tenir compte des largeur et longueur effectives des différentes zones. Ainsi on appellera « bord longitudinal » de la zone arrière, le bord qui fait partie du bord longitudinal de l'article.

Le qualificatif « interne » désigne les faces qui sont dirigées vers la peau de l'utilisateur. L'utilisateur peut être un bébé ou un adulte souffrant d'incontinence. Il a été trouvé toutefois que les articles selon l'invention étaient particulièrement avantageux dans le cas des bébés.

La feuille perméable aux liquides au moins dans la zone centrale peut être, notamment, une feuille de non-tissé. Il est également possible de placer, sous ce non-tissé, deux demi-bandes imperméables laissant dégagée une ouverture centrale, comme décrit dans la demande de brevet WO 83/03051 de la société déposante.

Les éléments d'attache situés sur la zone de tronc ainsi qu'aux extrémités de la ceinture sont du type à boucles ou à griffes. Ce dispositif est mieux connu sous le nom de marque « Velcro » (R). Mais il est bien évident que l'invention n'est pas strictement limitée à ce type d'élément. Elle comprend de manière générale tous les éléments d'attache qui, fixés, présentent une forte résistance au cisaillement et une faible résistance au pelage.

Quand les premiers éléments d'attache sont « à boucles » les seconds éléments d'attache situés aux extrémités de la ceinture sont « à griffes ». De même, lorsque les premiers éléments d'attache sont « à griffes », les seconds sont « à boucles ». Cette dernière solution est préférée dans le cas de la présente invention.

Ces éléments d'attache sont dimensionnés de telle façon qu'ils permettent un réglage du positionnement et, ainsi, une excellente adaptation de l'article à l'utilisateur.

De préférence encore, les premiers éléments d'attache sont placés à une distance du bord latéral supérieur de la zone de tronc telle que ledit bord puisse être replié vers l'intérieur pour améliorer l'étanchéité du tour de taille.

Afin d'assurer une meilleure étanchéité de l'article au porter, deux laminettes élastiques sont présentes au niveau de la zone d'entrejambe, comme décrit par exemple dans le brevet FR-A-2 011 778 de la Société déposante et les premiers éléments d'attache sont positionnés à l'extérieur et de part et d'autre de la surface longitudinale délimitée par des laminettes élastiques (voir leur prolongement fictif) et les bords latéraux extrêmes de l'article.

Les forces de friction doivent être suffisamment importantes pour permettre d'éviter le glissement de la ceinture sur face externe de la zone de tronc. Ceci peut être obtenu de plusieurs façons :

La ceinture peut être elle-même antidérapante. Elle sera avantageusement formée de matériaux filamenteux longitudinaux dont une partie est constituée de fils de gomme, ces derniers étant de préférence associés en torsade.

La friction peut également être obtenue par un traitement approprié, au moyen d'un produit antidérapant, de la surface externe de la zone de tronc venant en contact avec la ceinture et/ou la zone de la ceinture venant en contact avec cette zone de tronc. Parmi les produits antidérapants utilisables, on peut citer ceux qui permettent une enduction. L'enduction peut être continue ou discontinue par plages ou par traits. Cette seconde technique est préférée. Les produits antidérapants peuvent être déposés notamment à proximité des bords longitudinaux de la ceinture et/ou de la zone tronc venant en contact avec la ceinture. Une autre variante avantageuse prise en combinaison ou non avec la précédente consiste en ce que les produits antidérapants sont déposés selon des lignes longitudinales sur la ceinture.

Parmi les produits autorisant une enduction on peut citer les adhésifs durcissant à chaud et à faible tack comme les émulsions acryliques. Toutefois, on préférera les produits non sensibles à la pression, durcissant à froid, plus connus sous le qualificatif « thermofusibles » (en anglais « Hot-Melt »).

Un moyen également pour obtenir des forces de friction importantes et améliorer le maintien réciproque de la ceinture et de la zone de tronc externe venant en contact avec ladite ceinture consiste à les pourvoir respectivement d'éléments d'attache complémentaires.

De préférence alors le ou les éléments d'attache situés sur la zone de tronc externe en contact avec la ceinture sont identiques aux éléments d'attache situés sur l'autre zone de tronc. Ceci présente l'avantage de pouvoir utiliser une ceinture constituée d'une bande revêtue sur toute une face d'éléments d'attache complémentaires. Il sera préférable alors que les éléments d'attache situés sur la ceinture soient à boucles, les éléments d'attache situés sur la zone de tronc étant à griffes. De cette manière la ceinture ne présente aucun moyen d'accrochage risquant de se fixer sur les vêtements du bébé. Une réalisation avantageuse consiste à prévoir deux éléments d'attache sur la zone de tronc en contact avec la ceinture et lorsque ceux-ci sont situés symétriquement par rapport à l'axe longitudinal du change on obtient un maintien très efficace de la zone de tronc.

Pour améliorer encore ce maintien il est possible de traiter les volets extrêmes de la zone de tronc en contact avec la ceinture avec un revêtement adhérent ayant une résistance au pelage faible ou nulle comme les colles thermodurcissables. Il convient de rappeler qu'une fois l'article d'hygiène mis en place sur le corps de l'utilisateur, ces volets extrêmes sont recouverts par les volets extrêmes correspondants de l'autre zone de tronc.

De préférence, la ceinture est extensible de manière à améliorer encore la fixation de la zone de tronc en contact avec la ceinture. L'allongement maximum de cette ceinture sera de préférence supérieur à 30 %. La ceinture peut consister en un copolymère-éthylène-acétate de vinyle, mais sera de préférence formée de matériaux filamenteux longitudinaux extensibles enfermés dans une grille plastique rigide. Cette structure confère à la ceinture une très bonne extensibilité longitudinale et une extensibilité transversale quasi nulle. Par ailleurs, les saillies formées par les matériaux filamenteux vis-à-vis du polyéthylène extérieur de la zone de tronc procurent un coefficient de friction élevé.

L'invention sera mieux comprise en se référant aux modes de réalisation particuliers illustrés par les dessins en annexe :

La figure 1 est une vue en plan de la face interne d'un change avec une ceinture séparée.

La figure 2 est une vue en section transversale selon AA du change de la figure 1.

La figure 3 est une vue en perspective d'un change avec la ceinture partiellement positionnée.

La figure 4 est une vue en perspective d'un change avec la ceinture positionnée.

La figure 5 est une vue en perspective d'un appareil sur lequel a été testée l'efficacité du traitement antidérapant.

Les figures 6 à 9 représentent le change selon quatre variantes de réalisation.

Le change 1 est confectionné selon l'enseignement de la demande de brevet française du déposant FR-A-8 203 409. Il est de forme sensiblement rectangulaire et comprend deux zones de tronc 2 et 4, et une zone d'entre-jambe 3 dont les bords présentent une échancrure arrondie ou trapézoïdale.

Ce change est constitué d'un matelas absorbant 5, formé de préférence par de la mousse de cellulose, éventuellement chargée par une poudre de « superabsorbant » telle que celle décrite par exemple dans le brevet US-A4 043 952.

Le matelas 5 est avantageusement formé de plusieurs couches successives dégageant un canal central, comme décrit dans la demande de brevet française FR-A-8 317 723 et est enveloppé d'une feuille de tissu-ouate 7. Il est recouvert sur sa face externe d'une feuille imperméable 6 telle que du polyéthylène et, sur sa face interne, partiellement recouvert de deux demi-feuilles 8 imperméables, comme du polyéthylène, découpées de façon à former une fenêtre centrale 9 permettant la pénétration de l'urine et elles-mêmes recouvertes d'une feuille de non-tissé 17. Entre les deux feuilles 6 et 8 soudées entre elles le long de leurs bords de part et d'autre du matelas absorbant 5, dans la zone d'entre-jambe 3, sont fixées, à l'état tendu, deux laminettes élastiques 10 permettant d'assurer une meilleure étanchéité du change lorsque celui-ci est positionné.

Au niveau de la zone arrière 4, sur la face externe, à proximité des bords longitudinaux et à une distance d'environ 5 cm du bord latéral supérieur sont positionnés deux éléments d'attaches à bouches « Velcro » (R) 11 en polyamide. Ces deux éléments d'attache 11 sont situés de part et d'autre de la surface délimitée par les laminettes élastiques 10 (voire leur prolongement fictif) et les bords latéraux extrêmes de l'article et sont suffisamment larges (2 à 5 cm de côté) pour permettre le réglage du positionnement de la ceinture 12, décrite ci-après.

La ceinture 12 est formée de préférence d'une bande en tissu élastique formée de matériaux filamenteux longitudinaux extensibles en LYCRA (R) par exemple, 14 pris dans un filet rigide 15 en matière plastique telle que du polyamide.

Aux extrémités de la ceinture 12 sont fixés par des moyens connus en soi, deux éléments d'attache « à griffe » « Velcro » (R) 16 complémentaires des éléments d'attache 11 « à boucles ».

Lors de la mise en place du change, un premier élément d'attache complémentaire 16 est fixé sur un élément d'attache 11, puis la zone de tronc arrière 4 est rabattue de manière à ce que les parties situées à proximité des bords longitudinaux recouvrent les bords de l'autre zone de tronc avant 2. Ensuite, le second élément d'attache complémentaire 16 est fixé sur l'élément d'attache 11, restant de manière à ce que la ceinture soit positionnée sur l'abdomen de l'utilisateur.

On peut également envisager un mode de mise en place de l'article tel que la ceinture soit positionnée au niveau des reins de l'utilisateur.

A titre indicatif les dimensions (en cm) d'un change pour bébé sont les suivantes :

| | |
|---|---|
| largeur des zones de tronc | 31 |
| longueur | 54 |
| distance des premiers éléments d'attache au bord supérieur latéral | 5 |
| distance des premiers éléments d'attache au bord longitudinal | 1 |
| longueur de la ceinture | 28 |
| allongement maximal de la ceinture | 67 % |

Conformément à l'invention pour obtenir un coefficient de friction important entre les surfaces en contact on incorpore dans la matière des éléments antidérapants, par exemple des fils de gomme associés en torsade aux filaments de la ceinture ou bien on traite la surface d'au moins l'une de ces zones en contact.

On a vérifié l'efficacité des traitements appliqués à la ceinture, d'une part au moyen d'un appareillage destiné à simuler les forces qui s'exercent sur le change en position, d'autre part, in vivo, sur des bébés en crèche.

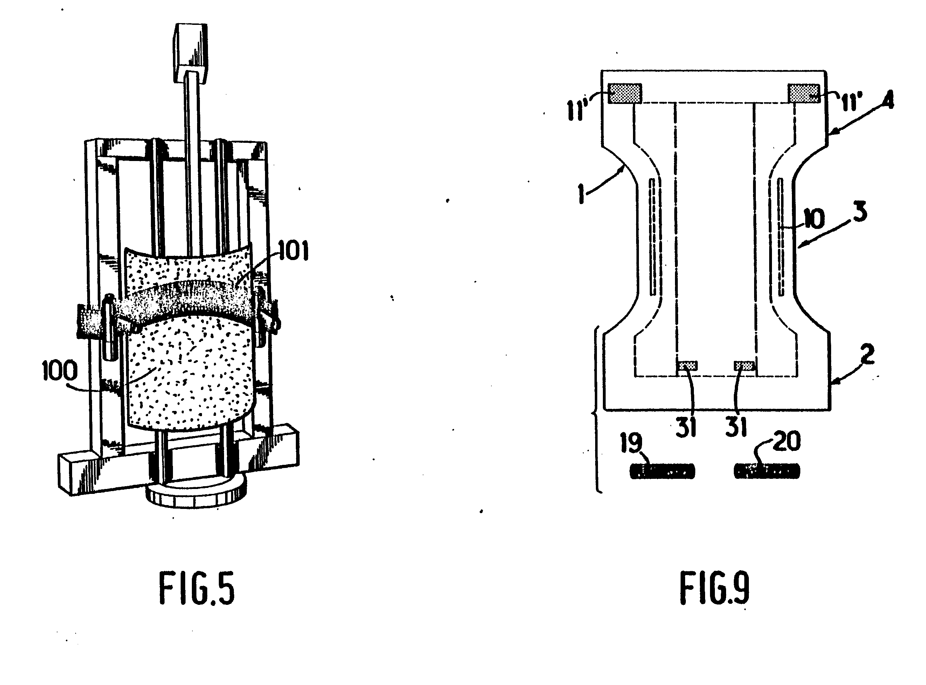

L'appareil permettant d'effectuer les tests est illustré par la figure 5.

Une feuille de polyéthylène (100) est collée sur une surface métallique incurvée mobile, reliée à un dynamomètre. Deux fixations à verrouillage excentrique permettent de maintenir la bande élastique (101) en contact avec le polyéthylène. La bande subit un allongement de 30 % puis on effectue 10 tractions avec une vitesse de déplacement de 150 mm/mn.

On mesure :

la force maximale moyenne Fm (en newton) — c'est-à-dire la force maximale limite au-delà de laquelle la ceinture glisse sur la feuille de polyéthylène.

L'énergie maximale Em (en Joule), mesurée par intégration. La valeur de l'énergie mesurée est très importante car selon que la ceinture lorsqu'elle commence à glisser « saute » ou au contraire glisse lentement, l'énergie dépensée sera différente. Cette mesure est donc liée au coefficient de frottement.

### Exemple 1

Sur la ceinture, on dépose 4 g/m linéaire de produit « thermofusible » « BERICOL 17 H 228 » (R).

Le dépôt est effectué par pistolettage sur le filet de polyamide selon quatre lignes longitudinales, symétriques deux à deux par rapport à l'axe longitudinal de la ceinture et situées à proximité des bords longitudinaux. Les deux dépôts extérieurs sont situés à environ 5 mm des bords longitudinaux respectifs.

Après durcissement du produit, on procède aux essais selon le mode opératoire décrit ci-dessus.

On obtient les résultats suivants :

| | |
|---|---|
| Fm | 21,6 N |
| Em | 2,8 J |

Des essais effectués sur 60 bébés de 12 à 18 kg en crèche révèlent un maintien parfait.

### Exemple 2

Selon le mode opératoire de l'exemple 1 on dépose par pistolettage une même quantité d'émulsion acrylique « ACRONAL 14D » (R) sur la ceinture.

On obtient les résultats suivants :

| | |
|---|---|
| Fm | 7,4 N |
| Em | 1,13 J |

Les essais en crèche révèlent un maintien qui, bien qu'acceptable, est moins satisfaisant que pour les produits thermofusibles.

### Exemple 3

Sur la ceinture on dépose 1,2 g/m linéaire de produit thermofusible Ferustick Réf. 73121Y (R) de la Société RMC Belix.

Le dépôt est effectué par pistolettage, par traits, sur le filet de polyamide selon quatre lignes longitudinales symétriques deux à deux par rapport à l'axe longitudinal de la ceinture et situées à proximité des bords longitudinaux. Les deux dépôts extérieurs sont situés à environ 5 mm des bords longitudinaux respectifs.

Après durcissement du produit on procède aux essais selon le mode opératoire décrit ci-dessus.

On obtient les résultats suivants :

| | |
|---|---|
| Fm | 18,7 N |
| Em | 2,2 J |

Des essais effectués sur 60 bébés de 12 à 18 kg en crèche révèlent un maintien parfait.

### Exemple 4

Avant confection de la ceinture on adjoint en torsade aux matériaux filamenteux de LYCRA (R) des fils de gomme.

On obtient les résultats suivants :

| | |
|---|---|
| Fm | 29,5 N |
| Em | 2,6 J |

Les essais en crèche révèlent un maintien parfait de la ceinture.

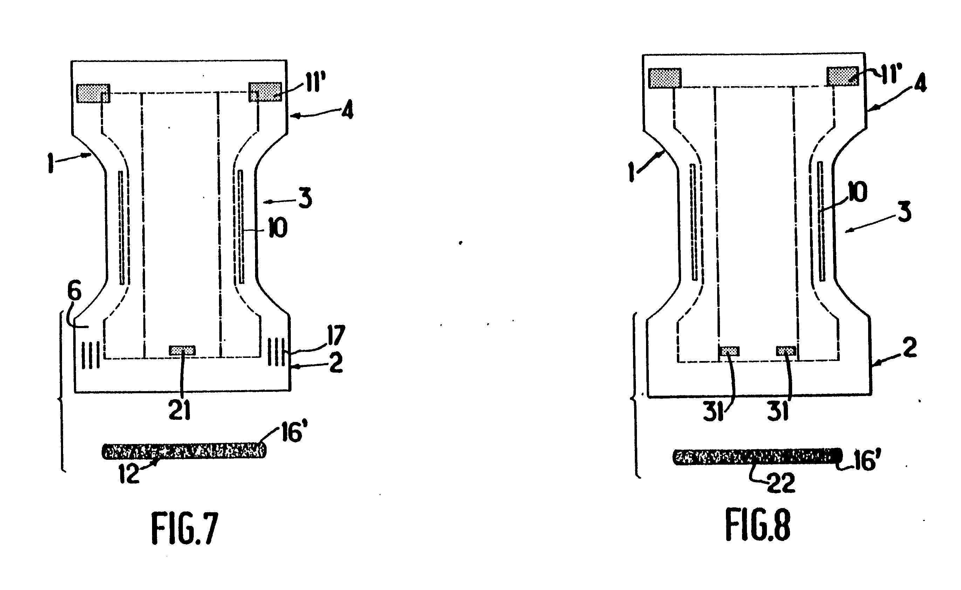

Les figures 6 à 9 se rapportent à des modes de réalisation de l'invention où le frottement important est assuré par des moyens d'attache du même type que les premiers et deuxièmes éléments d'attache.

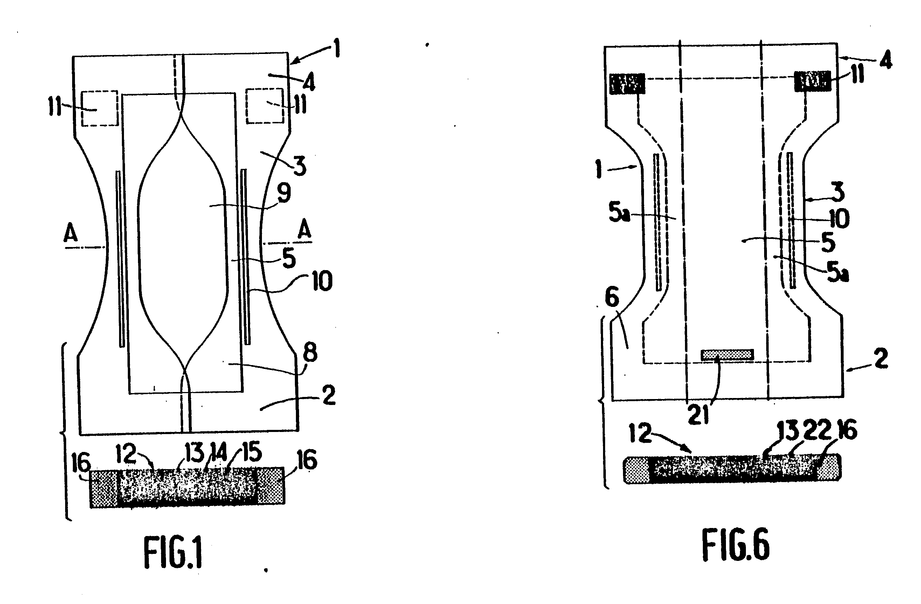

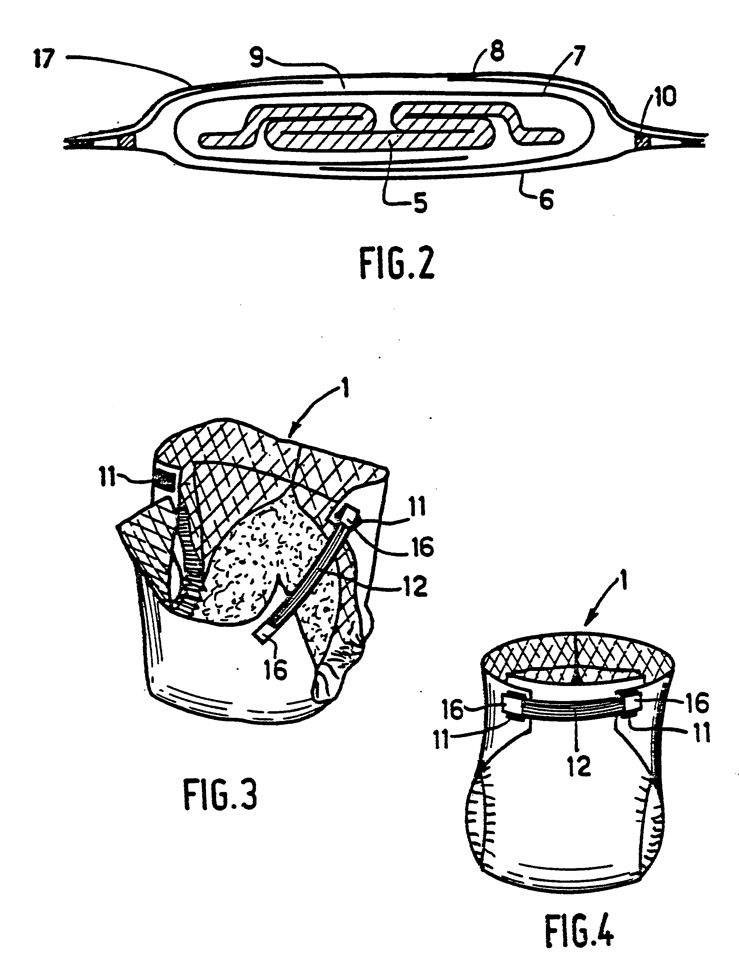

Sur la figure 6 le change est représenté du côté de la face externe, ses éléments semblables à ceux des figures 1 et 2 portent les mêmes références. Dans cet exemple le matelas 5 comprend deux parties latérales 5a qui débordent sur les cuisses du bébé.

Sur la zone avant externe 2, est positionné selon l'axe de symétrie du change et au niveau de la partie correspondante à l'extrémité latérale du matelas un élément d'attache 21 à griffes.

La ceinture 12 est formée d'une bande en tissu élastique munie d'éléments d'attache à boucles 22 sur une de ses faces dans la zone 13 venant en contact avec la zone avant 2. Aux extrémités de la ceinture 12 sont fixés par des moyens connus en soi, deux éléments d'attache 16 à griffes. Ainsi, lorsque le change est positionné sur le bébé, le fait que la ceinture elle-même soit constituée par un élément d'attache permet une mise en place sûre et rapide du change et un maintien efficace de la zone de tronc avant.

Le change décrit à la figure 7 est identique au change décrit ci-dessus sauf en ce qui concerne la nature des éléments d'attache :

Les éléments d'attache 11' situés sur la zone de tronc arrière 4 sont à griffes tandis que les éléments d'attache 16' situés aux extrémités de la ceinture 12 sont à boucles. Dans ce cas la ceinture et les extrémités sont formées d'une seule bande élastique à boucles.

Par ailleurs, les volets latéraux extrêmes de la zone de tronc avant 2 sont traités selon des lignes 17 par une colle ayant une faible valeur de résistance au pelage comme les colles durcissables à chaud dites en anglais « hot-melt » de manière à améliorer encore le maintien de la zone de tronc avant lorsque le change est positionné.

Le change représenté sur la figure 8 est identique au change décrit à la figure 7 sauf en ce qui concerne le nombre d'éléments d'attache :

Au lieu d'un seul élément d'attache situé sur la zone avant au centre, sont présents deux éléments d'attache 31 analogues mais situés, au même niveau par rapport au matelas absorbant, de part et d'autre de l'axe longitudinal du change, toujours dans la partie médiane du matelas : cette disposition assure encore une meilleure stabilité de la zone de tronc avant.

Lors de la mise en place de ce change, une des extrémités 16' de la ceinture 12 est fixée sur un élément d'attache 11' puis la zone avant est rabattue de manière à ce que la ceinture 12 soit fixée sur l'autre élément d'attache 11' et sur les deux éléments d'attache 31.

Le change représenté sur la figure 9 est une variante de celui représenté sur la figure 8 en ce qui concerne la structure de la ceinture.

Cette dernière est constituée de deux éléments identiques 19, 20 découpés dans une bande élastique à boucles. Lors de la mise en place du change les extrémités de la demi-ceinture 19 sont fixées sur les éléments d'attache 11', 31 situés d'un même côté du change et les extrémités de la demi-ceinture 20 sont fixées sur les éléments d'attache 11', 31 situés de l'autre côté du change.

## Revendications

1. Article d'hygiène à jeter (1) constitué :

a) d'un matelas absorbant (5) de forme sensiblement rectangulaire comportant deux zones de tronc (2) et (4) et une zone d'entre-jambe (3) recouvert sur sa face externe d'une feuille (6) imperméable aux liquides, telle qu'une feuille de polyéthylène et sur sa face interne d'une feuille (8) perméable aux liquides au moins au niveau de la zone centrale (9), ces deux feuilles étant partiellement ou totalement soudées entre elles le long de leurs bords longitudinaux, un premier élément d'attache (11, 11') étant apposé à proximité de chaque bord longitudinal sur la face externe au niveau d'une zone de tronc (4)

b) d'un moyen de fixation associé de manière amovible, comprenant une ceinture (12) pourvue à chaque extrémité d'un second élément d'attache complémentaire (16, 16') du premier élément d'attache (11, 11')
caractérisé en ce que, l'autre zone de tronc (4) externe et/ou au moins une zone (13) de la ceinture venant en contact avec cette zone de tronc externe est constituée de manière à posséder un coefficient de friction suffisant tel que, en position, l'article soit maintenu d'une part par la coopération des premiers éléments d'attache avec les seconds éléments d'attache, d'autre part par le frottement, l'une sur l'autre de la zone de tronc (4) et de ladite zone (13) de ceinture.

2. Article selon la revendication 1, caractérisé en ce que les premiers éléments d'attache (11) situés sur la face externe de la zone de tronc (4) sont à « boucles » et les seconds (16) « à griffes ».

3. Article à jeter selon l'une des revendications précédentes, caractérisé en ce que les éléments d'attache (11, 11') sont situés à une distance du bord latéral supérieur de la zone de tronc (4) telle qu'en position celui-ci puisse être replié vers l'intérieur pour améliorer l'étanchéité autour de la taille.

4. Article à jeter selon l'une des revendications précédentes, caractérisé en ce que de part et d'autre du matelas absorbant (5) dans la zone d'entre-jambe (3) sont fixées deux laminettes élastiques (10) et en ce que les premiers éléments d'attache (11, 11') sont positionnés à l'extérieur de la surface délimitée par les laminettes élastiques (10) — voire leur prolongement fictif — et les bords latéraux extrêmes de l'article.

5. Article à jeter selon l'une des revendications précédentes, caractérisé en ce que la ceinture (12) est extensible.

6. Article à jeter selon l'une des revendications précédentes, caractérisé en ce que la ceinture (12) est formée de matériaux filamenteux (14) longitudinaux auxquels sont associés des fils de gomme, par exemple en torsade.

7. Article à jeter selon la revendication 6, caractérisé en ce que les matériaux filamenteux longitudinaux sont enfermés dans une grille plastique rigide (15).

8. Article d'hygiène à jeter selon l'une des revendications 1 à 5, caractérisé en ce que la zone de tronc externe venant en contact avec la ceinture et/ou la zone (13) de la ceinture venant en contact avec cette zone de tronc sont traitées, au moins en partie, au moyen d'un produit antidérapant.

9. Article selon la revendication 8, caractérisé en ce que le produit antidérapant est déposé par enduction.

10. Article selon la revendication 9, caractérisé en ce que le produit antidérapant est non sensible à la pression, thermofusible et durcissable à froid.

11. Article selon l'une des revendications 9 ou 10, caractérisé en ce que l'enduction est discontinue par plages ou par traits.

12. Article selon l'une des revendications 9 à 11, caractérisé en ce que le produit antidérapant est déposé à proximité des bords longitudinaux de la ceinture et/ou de la zone de tronc externe venant en contact avec la ceinture.

13. Article selon la revendication 1, caractérisé en ce que le produit antidérapant est à base de fils de gomme.

14. Article à jeter selon l'une des revendications 8 à 12, caractérisé en ce que le produit antidérapant est déposé selon des lignes longitudinales sur la ceinture.

15. Article à jeter selon la revendication 14, caractérisé en ce que le produit antidérapant est extensible.

16. Article d'hygiène à jeter selon l'une des revendications 1 à 5, caractérisé en ce que la zone (13) de la ceinture en contact avec la zone de tronc externe est pourvue d'au moins un élément d'attache (22) et en ce que ladite zone de tronc externe (2) est munie d'au moins un élément d'attache (21) complémentaire dudit élément (22).

17. Article d'hygiène selon la revendication 16, caractérisé en ce que le ou les éléments d'attache (21) sont identiques aux éléments d'attache (11, 11') situés sur l'autre zone de tronc (4).

18. Article d'hygiène selon l'une des revendications 16 ou 17, caractérisé en ce que le ou les éléments d'attache situés sur la ceinture sont à boucles.

19. Article d'hygiène selon l'une des revendications 16 à 18, caractérisé en ce que deux éléments d'attache (31) sont présents sur la zone de tronc (2) en contact avec la ceinture.

20. Article d'hygiène selon la revendication 19, caractérisé en ce que les éléments d'attache (31) sont présents de part et d'autre et symétriquement par rapport à l'axe longitudinal du change.

21. Article d'hygiène selon les revendications 16, 19 et 20, caractérisé en ce que la ceinture (12) est constituée de deux éléments identiques (19, 20).

22. Article d'hygiène selon l'une des revendications précédentes, caractérisé en ce que les volets latéraux de la zone de tronc 2 en contact avec la ceinture sont traités avec un revêtement adhérent ayant une résistance au pelage nulle ou très faible.

## Claims

1. Disposable article of hygiene (1) consisting :
   a) of an absorbent pad (5) of substantially rectangular shape comprising two trunk regions (2) and (4) and a crotch region (3) covered on its outer face with a sheet (6) which is impervious to fluids, such as a sheet of polyethylene and on its inner face with a sheet (8) which is permeable to fluids at least in the central region (9), these two sheets being partially or completely welded together along their lengthwise edges, a first fastening member (11, 11') being appended near each lengthwise edge on the outer face in a trunk region (4)
   b) of a securing means associated in a removable manner, comprising a belt (12) provided at each end with a second fastening member (16, 16') complementing the first fastening member (11, 11')
characterized in that the other outer trunk region (4) and/or at least one region (13) of the belt coming into contact with this outer trunk region is made so as to have a sufficient coefficient of friction such that, when in position, the article is held, on the one hand, by the interaction of the first fastening members with the second fastening members and, on the other hand, by the reciprocal friction of the trunk region (4) and of the said belt region (13).

2. Article according to Claim 1, characterized in that the first fastening members (11) situated on the outer face of the trunk region (4) are provided with « loops » and the second members (16) « with hooks ».

3. Disposable article according to either of the preceding claims, characterized in that the fastening members (11, 11') are situated at a distance from the upper side edge of the trunk region (4) such that when in position this can be folded back inwards to improve the leakproofing around the waist.

4. Disposable article according to one of the preceding claims, characterized in that two elastic striplets (10) are attached on each side of the absorbent pad (5) in the crotch region (3) and that the first fastening members (11, 11') are positioned outside the area bounded by the elastic striplets (10) — or their imaginary extension — and the outermost side edges of the article.

5. Disposable article according to one of the preceding claims, characterized in that the belt (12) is stretchable.

6. Disposable article according to one of the preceding claims, characterized in that the belt (12) is made of lengthwise filamentous materials (14) with which rubber threads are associated, for example as a cable stitch.

7. Disposable article according to Claim 6, characterized in that the lengthwise filamentous materials are enclosed in a rigid plastic grid (15).

8. Disposable article of hygiene according to one of Claims 1 to 5, characterized in that the outer trunk region coming into contact with the belt and/or the belt region (13) coming into contact with this trunk region are treated, at least partly, with an antiskid product.

9. Article according to Claim 8, characterized in that the antiskid product is deposited by coating.

10. Article according to Claim 9, characterized in that the antiskid product is insensitive to pressure, of the hot-melt type and capable of setting when cold.

11. Article according to either of Claims 9 and 10, characterized in that the coating is discontinuous in the form of steps or lines.

12. Article according to one of Claims 9 to 11, characterized in that the antiskid product is deposited near the lengthwise edges of the belt and/or of the outer trunk region coming into contact with the belt.

13. Article according to Claim 1, characterized in that the antiskid product is based on rubber threads.

14. Disposable article according to one of Claims 8 to 12, characterized in that the antiskid product is deposited along lengthwise lines on the belt.

15. Disposable article according to Claim 14, characterized in that the antiskid product is stretchable.

16. Disposable article of hygiene according to one of Claims 1 to 5, characterized in that the region (13) of the belt in contact with the outer trunk region is provided with at least one fastening member (22) and in that the said outer trunk region (2) is equipped with at least one fastening member (21) complementary to the said member (22).

17. Article of hygiene according to Claim 16, characterized in that the fastening member or members (21) is, or are, identical to the fastening members (11, 11') situated on the other trunk region (4).

18. Article of hygiene according to either of Claims 16 and 17, characterized in that the fastening member or members situated on the belt is, or are, looped.

19. Article of hygiene according to one of Claims 16 to 18, characterized in that two fastening members (31) are present on the trunk region (2) in contact with the belt.

20. Article of hygiene according to Claim 19, characterized in that the fastening members (31) are present on each side and symmetrically in relation to the lengthwise axis of the change.

21. Article of hygiene according to Claims 16, 19 and 20, characterized in that the belt (12) consists of two identical members (19, 20).

22. Article of hygiene according to one of the preceding claims, characterized in that the side flaps of the trunk region 2 in contact with the belt are treated with an adhesive coating which has very low or no peel strength.

**Patentansprüche**

1. Hygienischer Wegwerfartikel (1), bestehend aus :
   a) einer im wesentlichen rechteckigen absorbierenden Schicht (5) mit zwei Basisbereichen (2 und 4) und einem Zwickelbereich (3), der auf seiner Außenseite mit einer flüssigkeitsundurchlässigen Folie (6), z. B. einer Polyäthylenfolie, und auf seiner Innenseite zumindest im zentralen Bereich (9) mit einer flüssigkeitsdurchlässigen Folie (8) versehen ist, wobei diese beiden Folien teilweise oder ganz entlang ihrer Längsränder miteinander verschweißt sind, wobei ein erstes Befestigungselement (11, 11') in der Nähe jedes Längsrandes an der Außenseite auf Höhe eines Bassibereichs (4) angebracht ist,
   b) abnehmbaren Befestigungsmitteln in Form eines Gürtels (12), der an jedem Ende mit einem zum ersten Befestigungselement (11, 11') komplementären zweiten Befestigungselement (16, 16') versehen ist,
dadurch gekennzeichnet, daß der andere äußere Basisbereich (4) und/oder zumindest ein mit diesem äußeren Basisbereich in Berührung stehender Bereich (13) des Gürtels so ausgebildet ist, daß er einen ausreichenden Reibbeiwert besitzt, damit der Artikel in seiner Lage gehalten wird einerseits durch Zusammenwirken der ersten Befestigungselemente mit den zweiten Befestigungselementen und andererseits durch den Reibschluß zwischen dem Basisbereich (4) und dem besagten Bereich (13) des Gürtels.

2. Wegwerfartikel nach Anspruch 1, dadurch gekennzeichnet, daß die auf der Außenseite des Basisbereiches (4) angeordneten Befestigungselemente (11) « ösenartig » und die zweiten Befestigungselemente (16) « hakenartig » ausgebildet sind.

3. Wegwerfartikel nach einem der vorhergehenden Ansprüche, dadurch gekennzeichnet, daß die Befestigungselemente (11, 11') in einem Abstand zu dem oberen Seitenrand des Basisbereiches (4) so angeordnet sind, daß dieser zur Verbesserung der Dichtheit um die Taille herum nach innen umgeschlagen werden kann.

4. Wegwerfartikel nach einem der vorhergehenden Ansprüche, dadurch gekennzeichnet, daß beidseitig zu der absorbierenden Schicht (5) in dem Zwickelbereich (3) zwei elastische Streifen (10) befestigt sind und daß die ersten Befestigungselemente (11, 11') außerhalb derjenigen Fläche angeordnet sind, die von den elastischen Streifen (10) — gesehen in ihrer gedachten Verlängerung — und den äußersten Seitenrändern des Artikels begrenzt wird.

5. Wegwerfartikel nach einem der vorhergehenden Ansprüche, dadurch gekennzeichnet, daß der Gürtel (12) verlängerbar ist.

6. Wegwerfartikel nach einem der vorhergehenden Ansprüche, dadurch gekennzeichnet, daß der Gürtel (12) aus Längsfaserstoffen (14) gebildet ist, denen beispielsweise gekordelte Gummifäden zugeordnet sind.

7. Wegwerfartikel nach Anspruch 6, dadurch gekennzeichnet, daß die Längsfaserstoffe in einem starren Kunststoffgitter (15) eingeschlossen sind.

8. Wegwerfartikel nach einem der Ansprüche 1 bis 5, dadurch gekennzeichnet, daß der mit dem Gürtel in Berührung stehende äußere Basisbereich und/oder der mit diesem Basisbereich in Berührung stehende Bereich (13) des Gürtels zumindest teilweise mit einem rutschfesten Produkt behandelt sind.

9. Wegwerfartikel nach Anspruch 8, dadurch gekennzeichnet, daß das rutschfeste Produkt durch Beschichten aufgebracht ist.

10. Wegwerfartikel nach Anspruch 9, dadurch gekennzeichnet, daß das rutschfeste Produkt nicht druckempfindlich, thermoschweißbar und kältehärtbar ist.

11. Wegwerfartikel nach Anspruch 9 oder 10, dadurch gekennzeichnet, daß die Beschichtung bereichs- oder linienweise unterbrochen ist.

12. Wegwerfartikel nach einem der Ansprüche 9 bis 11, dadurch gekennzeichnet, daß das rutschfeste Produkt in der Nähe der Längsränder des Gürtels und/oder des mit dem Gürtel in Berührung stehenden äußeren Basisbereiches aufgebracht ist.

13. Wegwerfartikel nach Anspruch 1, dadurch gekennzeichnet, daß das rutschfeste Produkt ein Produkt auf der Basis von Gummifäden ist.

14. Wegwerfartikel nach einem der Ansprüche 8 bis 12, dadurch gekennzeichnet, daß das rutschfeste Produkt auf dem Gürtel entlang der Längslinien aufgebracht ist.

15. Wegwerfartikel nach Anspruch 14, dadurch gekennzeichnet, daß das rutschfeste Produkt verlängerbar ist.

16. Wegwerfartikel nach einem der Ansprüche 1 bis 5, dadurch gekennzeichnet, daß der mit dem äußeren Basisbereich in Berührung stehende Bereich (13) des Gürtels mit mindestens einem Befestigungselement (22) versehen ist und daß der besagte äußere Basisbereich (2) mit mindestens einem zu dem Befestigungselement (22) komplementären Befestigungselement (21) versehen ist.

17. Wegwerfartikel nach Anspruch 16, dadurch gekennzeichnet, daß das Befestigungselement bzw. die Befestigungselemente (21) identisch sind zu den auf dem anderen Basisbereich (4) angeordneten Befestigungselementen (11, 11').

18. Wegwerfartikel nach Anspruch 16 oder 17, dadurch gekennzeichnet, daß das Befestigungselement bzw. die Befestigungselemente, die am Gürtel angeordnet sind, Ösen bzw. Schnallen sind.

19. Wegwerfartikel nach einem der Ansprüche 16 bis 18, dadurch gekennzeichnet, daß zwei Befestigungselemente (31) auf dem mit dem Gürtel in Berührung stehenden Basisbereich (2) vorhanden sind.

20. Wegwerfartikel nach Anspruch 19, dadurch gekennzeichnet, daß die Befestigungselemente (31) beidseitig und symmetrisch zur Wechsel-Längsachse angeordnet sind.

21. Wegwerfartikel nach den Ansprüchen 16, 19 und 20, dadurch gekennzeichnet, daß der Gürtel (12) von zwei identischen Teilen (19, 20) gebildet wird.

22. Wegwerfartikel nach einem der vorhergehenden Ansprüche, dadurch gekennzeichnet, daß die Seitenflügel des mit dem Gürtel in Berührung stehenden Basisbereiches (2) mit einem haftenden Überzug behandelt sind, der keinen oder einen sehr schwachen Abziehwiderstand hat.

**FIG.1**

**FIG.6**

0 169 868

**FIG.2**

**FIG.3**

**FIG.4**

FIG.9

FIG.5

3

FIG.7

FIG.8

0 169 868